# EUROPEAN PATENT SPECIFICATION

(11) **EP 4 117 638 B1**
(45) Date of publication and mention of the grant of the patent: **06.05.2026**
(21) Application number: 21718645.1
(22) Date of filing: 09.03.2021
(51) Int. Cl.: A61K 9/16, A61K 9/20, A61K 9/28, A61K 31/472, A61P 1/06, A61P 1/16, A61P 13/06, A61P 13/10, A61P 13/12, A61P 15/02

(54) **CONTROLLED RELEASE FORMULATIONS COMPRISING DROTAVERINE OR SALT THEREOF**
FORMULIERUNGEN MIT KONTROLLIERTER FREISETZUNG MIT DROTAVERIN ODER EINEM SALZ DAVON
FORMULATIONS À LIBÉRATION CONTRÔLÉE COMPRENANT DE LA DROTAVÉRINE OU UN SEL DE CETTE DERNIÈRE

(30) Priority: 09.03.2020 IN 202011010072
(43) Date of publication of application: 18.01.2023
(73) Proprietor: Drotastar LLC, Lewes, County of Sussex, DE 19958 (US)
(72) Inventor: BERLIA, Sushma Paul, New Delhi Delhi 110017 (IN); BERLIA, Nishant, New Delhi Delhi 110011 (IN); SINGH, Gurvinder, New Delhi Delhi 110058 (IN); BHANDARI, Sunder Singh, Noida Uttar Pradesh 201301 (IN); DIWAN, Anupama, Sona Haryana 122103 (IN)
(74) Representative: Finnegan Europe LLP
(86) International application number: PCT/IB2021/051942
(87) International publication number: WO 2021/181262

(56) References cited:
- EP-A1- 3 520 781
- EP-A2- 1 200 088
- EP-B1- 1 200 088
- WO-A1-2016/075617

## Description

### FIELD OF THE INVENTION

In some aspects, the present invention relates to controlled release formulations comprising Drotaverine or salt thereof or similar active agents those are prone to oxidative and hydrolytic degradation and methods of preparation thereof. In some aspects, the invention relates to treatment of symptoms of gastrointestinal, biliary, urological and/or gynaecological disorders characterized by spastic conditions of smooth muscles by administering the controlled release formulations of the present invention.

### BACKGROUND OF THE INVENTION

It is well known in the field of art to prepare formulations which provide controlled release of pharmacologically active substances on oral administration to humans and animals. Controlled release (CR) formulations decrease the frequency of administration required to maintain therapeutically effective plasma drug levels. In addition, by producing more constant blood levels, such formulations can reduce the large changes in plasma levels observed between doses. Controlled release formulations are intended to provide a longer period of pharmacological action after administration than is ordinarily achieved after administration of immediate-release (IR) dosage forms. Such longer periods of response provide therapeutic benefits that are not achieved by immediate release preparations. Furthermore, controlled release preparations result in better patient compliance.

Drotaverine HCl [(Z)-1-(3,4-diethoxybenzylidene)-6,7-diethoxy-1,2,3,4-tetrahydroisoquinoline] is a benzylisoquinoline derivative, and is an analogue of papaverine.

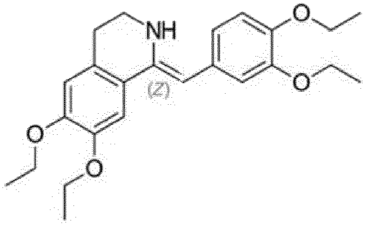

The molecular weight of Drotaverine is 397.50 g/mol and the Empirical Formula is C₂₄H₃₁NO₄. Drotaverine is a potent spasmolytic which acts directly on the smooth muscles by inhibiting phosphodiesterase activity and is devoid of any anticholinergic side effects. Drotaverine is a selective inhibitor of phosphodiesterase isoenzyme IV and has been found useful in controlling spastic disorders of smooth muscle. Drotaverine has a dual mechanism of action. Firstly, it inhibits enzyme phosphodiesterase IV (PDE IV). This causes an increase in cyclic adenosine monophosphate level (cAMP) in smooth muscles, resulting in a decrease in concentration of free calcium ions (Ca²⁺). Secondly, it inhibits calmodulin (CM), which is a calcium binding protein. The decrease in Ca²⁺ ions along with the inhibition of calmodulin leads to inhibition of "Calmodulin-Calcium complex" (CM-Ca²⁺) formation. This process inhibits myosin light chain kinase enzyme (MLCK), leading to dephosphorylation of the actomyosin phosphate complex and thus causing normalization of smooth muscles.

Drotaverine is a highly potent and safe antispasmodic (spasmolytic) agent, suitable for oral and parenteral administration. Drotaverine is currently marketed as a conventional IR product indicated in spasmodic pain of smooth muscles. It relieves and prevents smooth muscle spasm of various organs regardless of their function and innervation. Primarily, the drug alone and in combination is indicated in the treatment of various gastrointestinal, biliary, urological and/or gynecological disorders characterized by spastic conditions of smooth muscles.

For example, Drotaverine is indicated in:
i) spastic conditions of the gastrointestinal tract: irritable bowel syndrome, biliary colics and spastic conditions of the biliary tract such as cholecystolithiasis, cholecystitis, cholangitis.
ii) renal colics and spastic conditions of the urogenital tract: nephrolithiasis, ureterolithiasis, pyelitis, cystitis.
iii) spastic conditions of the uterus: dysmenorrhoea, imminent abortion, uterine tetanus.

It is also widely used for augmentation of labor, in the therapy of spastic pain due to gastric and duodenal ulcer and prior to instrumental diagnostic procedures. The approved daily doses are 120-240 mg (in 2-3 divided doses) in adults, 40-120 mg (in 2-3 divided doses) in children between 1-6 years of age and 80-200 mg (in 2-5 divided doses) in children over 6 years. Due to its non-anticholinergic nature, Drotaverine can safely be prescribed for a long duration. Drotaverine has been registered in more than 50 countries, in Europe, Asia, Africa and Central America.

In indications like irritable bowel syndrome (IBS), where pain is the predominant symptom, antispasmodics have been and remain the main agents for therapy. Drotaverine exhibits a normalizing effect on intestinal smooth muscle, which helps in alleviating pain and does not have side effects like anticholinergics. Anticholinergic antispasmodics are avoided in IBS where constipation is present (IBS-Constipation is a predominant symptom).

Drotaverine brings relief of spasm in IBS by its unique site-specific action and causes normalization of smooth muscle contraction. Thus, it does not cause constipation, making it suitable for use in all types of IBS (Diarrhea predominant IBS, Constipation predominant IBS, and mixed IBS). The antispasmodic Drotaverine has long been used to relieve pain in Irritable bowel syndrome (IBS). However, there remains an issue that the drug must be administered 2-3 times a day, which may lead to poor patient compliance and inconvenience to the patients.

Abdominal pain is a common problem in children. A variety of disorders can cause acute abdominal pain in children. Drotaverine is an effective and safe pharmaceutical agent in the management of recurrent abdominal pain of childhood. It is the most commonly used off-label medication for alimentary tract problems in preschool- and school-aged children.

Drotaverine was associated with improved progress of the first stage of labor without increasing the feto-maternal compromise among nulliparous women. Drotaverine can be considered as an effective and safe pharmaceutical agent to shorten the duration of the first stage of spontaneous labor among nulliparous women.

Drotaverine is efficient and advantageous in ameliorating and/or treating benign prostatic hyperplasia, urinary disorders, disorders related to bladder dysfunction, lower urinary tract symptoms associated or not associated with benign prostatic hyperplasia, urinary incontinence, bladder outlet obstruction associated or not associated with benign prostatic hyperplasia, interstitial cystitis and overactive bladder.

Drotaverine contributes very effectively either alone or in combinations in relieving painful spasm in a wide variety of disease of gastroenterological, urological, gynecological and/or obstetrical origin and is the first drug of choice in emergency medicine. Drotaverine does not mask the symptoms of "*acute abdomen*" and this feature of Drotaverine makes it unique.

A spasmolytic like Drotaverine and anti-prostaglandin like mefenamic acid may be used as analgesia in women undergoing IUD (intra-uterine device) insertion in the clinic. Drotaverine allays early-onset pain and potentiates a sustained analgesic effect of mefenamic acid. The combination of Drotaverine and mefenamic acid is effective in decreasing the pain during the procedure and its effect lasts longer than that of paracervical block or intravenous sedation.

Addition of Drotaverine to Aceclofenac provides significant therapeutic benefits in relieving pain associated with primary dysmenorrhoea. Drotaverine produces rapid pain relief due to antispasmodic action while Aceclofenac provides sustained analgesic effect. The fixed-dose immediate release (IR) combination of Aceclofenac-Drotaverine should therefore be considered as effective and well tolerated treatment for primary dysmenorrhoea.

There is a significant decrease in abdominal pain in subjects with acute infectious gastroenteritis when treated with Drotaverine hydrochloride (80 mg) and Paracetamol (PCM) 500 mg as compared to subjects treated with PCM alone, demonstrating the effectiveness of a fixed dose IR combination of Drotaverine hydrochloride (80 mg) and PCM (500 mg) for amelioration of abdominal pain associated with acute infectious gastroenteritis.
Some other possible combinations with Drotaverine have been studied in the literature:
WO2016075617 discloses that a combination of an analgesic and an anti-spasmodic agent such as ibuprofen (400 mg) and Drotaverine (80 mg) for treating menstrual and abdominal cramps alleviates the pain associated with menstruation and relieves abdominal cramps.
Zhu, J. *et al* (2017) discloses that a combined therapy of ketorolac, diclofenac, and Drotaverine is effective in the relief of acute renal colic, especially pain due to mid and proximal ureteric lithiasis and that this therapy was associated with reduced use of rescue analgesia.

All of these marketed combinations with Drotaverine are available as immediate release dosage forms and none of the combinations disclose controlled release preparations.

The pharmacokinetic parameters, such as elimination, half-life, plasma clearance, renal clearance and apparent volume of distribution of Drotaverine, are not influenced by the route of drug administration (Bolaji OO *et al.,* 1996). The pharmacokinetic parameters for immediate release (IR) Drotaverine Tablets 80 mg have been studied. The drug is mainly eliminated by non-renal routes since renal clearance accounted for only 0.31 +/- 0.13% of the total plasma clearance. The absolute bioavailability was variable and subject-dependent and ranged between 24.5-91% with a mean of 58.2 +/- 18.2% (mean +/- SD). It is suggested that high variation in bioavailability of Drotaverine HCl after oral administration results in significant inter-individual differences in therapeutic response to the drug. In pharmacokinetic and bioavailability studies, Drotaverine has shown rapid absorption viz., onset of action within 12 minutes after oral administration of 80 mg strength IR tablets and almost 90% of drug absorption from small intestine. The peak plasma levels were obtained after 1 to 3 hours while elimination half-life ranges from 7 to 11.95 hours. Drotaverine seemed to be very safe in toxicological studies as the reported oral LD50 for Drotaverine is >1000mg/Kg body weight and hence has a wide therapeutic index (Blasko G, 1996).

CN102149382 provides that Drotaverine in free form or in form of a salt is helpful in improving and/or treating benign prostatic hyperplasia, urinary disorders, conditions involving bladder dysfunction, lower urinary tract symptoms associated with or unrelated to benign prostatic hyperplasia, urinary incontinence, bladder outlet obstruction, interstitial cystitis, and overactive bladder associated with or unrelated to benign prostatic hyperplasia; wherein the preferred Drotaverine salt is Drotaverine HCl. The pharmaceutical compositions provided were suitable for administration by oral or parenteral routes and may be prepared further for sublingual, subcutaneous, intramuscular, intravenous, intradermal, topical or rectal administration. The pharmaceutical compositions is prepared by incorporation of pharmaceutical additive(s) in a ratio ranging from 1% by weight to 90% by weight based on the weight of the Drotaverine or its salt. Examples of excipients used in the preparation of solid pharmaceutical compositions include, for example, lactose, sucrose, starch, talc, cellulose, dextrin, kaolin, calcium carbonate, and the like.

When preparing a solid composition in the form of a tablet, the active component is mixed with excipients. The tablets may be coated with sucrose or other suitable materials, or they may be treated such that they have extended or delayed activity, and they continuously release a predetermined amount of the active ingredient. A preparation in the form of gelatin capsules is obtained by mixing the active ingredient with a diluent and by pouring the mixture obtained into soft or hard gelatin capsules. For the preparation of liquid compositions for oral administration, use of a conventional inert diluent such as water or a vegetable oil has been reported. The liquid compositions are disclosed to incorporate, in addition to the inert diluent, auxiliaries such as moistening agents, suspension aids, sweeteners, aromatics, colorants, and preservatives. The liquid composition obtained can be filled in capsules made of an absorbable material such as gelatin. The CN102149382 invention provides Examples of solvents or suspension mediums used for preparation of compositions for parenteral administration, viz., injections and suppositories include water, propylene glycol, polyethylene glycol, benzyl alcohol, ethyl oleate, lecithin and the like. Examples of base materials used for suppositories include, for example, cacao butter, emulsified cacao butter, lauric lipid, witepsol. When the compositions of CN102149382 are administered in humans by parenteral route or oral route, the daily dose of Drotaverine varied between 15 to 250 mg; and preferably between 120 to 240 mg. More preferably, the dose of Drotaverine HCl is 40 mg once to six times per day, or 80 mg, once to three times per day as immediate release dosage form. The effect of Drotaverine was studied on induced plateau of contraction in human isolated detrusor muscle. CN102149382 showed that Drotaverine induces a significant concentration-dependent relaxation of the human detrusor muscle contraction.

EP2709599 B1 relates to a stable immediate-release pharmaceutical composition of Drotaverine HCl for oral administration. The document provides a pharmaceutical composition in form of a soft capsule for oral administration comprising 5 to 30% (w/w) of Drotaverine HCl; at least 60% (w/w) of a liquid mixture of a non-ionic hydrophilic surfactant and a nonionic hydrophobic surfactant, wherein the non-ionic hydrophilic surfactant is polysorbate 80 and the non-ionic hydrophobic surfactant is propylene glycol monocaprylate; wherein the weight ratio of Drotaverine HCl to the liquid mixture of surfactant is from 1:3 to 1:7. EP2709599 B1 addresses a problem with gelatin-based compositions, which is an apparent fall in dissolution upon aging, attributed to the cross-linking of gelatin containing products. The cross-linking causes formation of a swollen, very thin, tough, rubbery, water-insoluble membrane, also known as pellicle. The pellicle acts as a barrier and restricts release of the drug. Drugs like Drotaverine or its pharmaceutically acceptable salts or hydrates thereof, have a tendency to react with gelatin and induce cross linking owing to which the possibility of fall in dissolution during stability studies is high. EP2709599 B1 provides that such cross linking of gelatin is overcome by addition of certain weak acids such as tartaric acid, citric acid or its combinations thereof in combination with glycine; in an amount of about 0.1 to 1.0% on wt. basis of the gelatin shall formula. Further, the document provides that the preferred gelatin composition for use in constructing soft gelatin capsules for use with the Drotaverine composition includes gelatin and a plasticizer(s) like propylene glycol, and sorbitol. The results reveal that 100% (w/w) of the Drotaverine HCl dissolved is released after 30 minutes for NoSpa^{®} tablet and after 45 minutes for a composition according to EP2709599 B1.

EP1200088A2 relates to an analgestic / antipyretic immediate release composition in tablet form comprising paracetamol and Drotaverine HCl optionally along with codeine phosphate as active ingredients in a mixture with one or more organic acid or potassium sorbate as stabilizers, one or more usually used pharmaceutical auxiliary materials. The commonly used fillers and vehicles employed were lactose, mannitol, sorbitol, gliders, e.g., magnesium stearate, stearic acid and talc, furthermore substances facilitating disintegration, e.g. poly(vinylpoly-pyrrolidone), starch, cellulose and pigments, e.g., iron(III)-oxides, or organic dyes. The pharmaceutical compositions of EP1200088A2 optionally employed saccharide type fillers and an organic acid as stabilizer. The quantity of decomposition products in the compositions of EP1200088A2 was found to be low even after long time under the conditions of accelerated and long term stability tests too. The tablets produced according to EP1200088A2 were reported to be stable from physical and chemical aspects alike.

Indian Patent Application 1625/DEL/2006, provides solid oral immediate release pharmaceutical composition containing two therapeutically active agents to be used as a spasmolytic and analgesic composition. The application provides that spasmolytic drugs such as Drotaverine are often prescribed with anti-inflammatory agents, whenever there is an inflammatory condition associated with the muscle spasms. The application discloses that the two drugs i.e., ketorolac tromethamine and Drotaverine HCl when formulated into a dosage form as a mixture undergo a certain amount of degradation. The application circumvented incompatibility between the drugs by avoiding contact between ketorolac and Drotaverine and formulating solid oral pharmaceutical composition as a bilayer tablet. The solid dosage form further comprises a pharmaceutically acceptable carrier, which comprises one or more of diluent(s), binder(s), dis-integrant(s), glidant(s) and lubricant(s).

RU2535049C1 discloses a process of preparing stabilized Drotaverine hydrochloride. In order to protect Drotaverine hydrochloride from oxidative decomposition/degradation, Drotaverine hydrochloride is crystallized in the presence of a stabilizing additive of a mixture of citric acid and its sodium salt in an amount of up to 1.0% of the weight of Drotaverine hydrochloride fed for crystallization. The document discloses a method for producing a stabilized substance of Drotaverine hydrochloride by introducing stabilizing additives, characterized in that the stabilizing additives are introduced into the reaction mass during the process of recrystallization of Drotaverine hydrochloride from a solution. RU2199308C1 discloses that stabilization of Drotaverine hydrochloride is carried out by introducing stabilizing additives such as sodium sulfite, ethyl alcohol etc. in the solution.

WO2019149917A1 (EP 3520781 A1 is a family member of this application) discloses immediate release pharmaceutical composition comprising, in one dosage form, metamizole, Drotaverine, and caffeine, characterized in that metamizole in the said composition is present in the form of a granulate comprising metamizole, and Drotaverine and caffeine are present outside the granulate. WO2019149917A1 provides that the combination of metamizole, Drotaverine, and caffeine is known for its strong analgesic effect and such a combination can be used in case of severe and persistent pain and fever. Additionally, the combination of an analgesic substance (metamizole) with an antispasmodic substance (Drotaverine) effectively relieves spastic pains occurring during migraine, menstruation, colic, etc. The third active substance (caffeine) in the composition aids by enabling faster action of the medicine and intensifies the analgesic effects of the other substances (metamizole and Drotaverine). All the above cited literature discloses the immediate release preparations and none of them are related to the controlled release dosage forms of Drotaverine.

Om Prakash *et al.,* 2013 reported the development of floating drug delivery system containing Drotaverine. The main objective of the study reported by Om Prakash was to develop floating tablets of Drotaverine HCl that prolong the gastric residence time with total floatation time greater than 24 hours. This study also discloses using a combination of hydrophilic (HPMC) and hydrophobic (carbopol-934P) polymers as essential components along with Sodium Carbonate and Citric acid as gas forming agents for ensuring 24h drug release profile. The formulation as per this study shows very slow dissolution with around 50% drug release in 8 hours; 75% drug release in 16 hours and around 99% dissolution in 24 hours. This is merely a research paper and appears to have no industrial applicability. The objective of this research paper was to increase gastric residence time and there is no disclosure of viable controlled release formulation. Based on teachings of this prior art, a person skilled in the art cannot develop a formulation of Drotaverine having controlled release profile of 12-24 hrs.

Drotaverine HCl is highly susceptible to oxidation. It undergoes oxidative degradation by oxidative decomposition to Drotaveraldine in neutral or alkaline media, whereas upon exposure to light, the molecule is degraded to perparin and then to perparaldin by dehydrogenation.

In compatibility studies, it has been found that the chemical stability of Drotaverine hydrochloride solid pharmaceutical compositions is significantly influenced by the moisture content of the tableting excipients, granules and tablets, and the chemistry of the composition. The available prior art did not give any clue to prepare controlled release dosage forms of drotaverine which are chemically stable.

The interaction of Drotaverine HCl with a number of tableting excipients like magnesium stearate (which is widely used in tableting) has been studied and it has been found that the degradation of Drotaverine HCl is greatly enhanced by its alkaline hydrolysis especially in presence of moisture. In addition, the chemical instability of Drotaverine hydrochloride is significantly influenced by the pH of the formulation, and the degradation rate was largely enhanced in the presence of magnesium stearate. The developed formulation of current invention surprisingly demonstrates good stability even in the presence of Magnesium stearate and hence successfully overcame the limitation of adding Magnesium Stearate as reported in prior art

There also is a need to control moisture content levels to ensure minimal degradation of Drotaverine HCl in the finished product. Additionally, Drotaverine HCl or similar type of drug molecules that are sensitive to oxygen may also require encapsulation or special coating or a microenvironment with inert gas e.g., nitrogen etc. in the finished product primary packing to further improve shelf-life.

Perhaps due to very high susceptibility of Drotaverine to oxidative and hydrolytic degradation, and other reasons, a controlled release (CR) formulation of Drotaverine was never considered possible or feasible.

The goal of any drug delivery system is to provide a therapeutic amount of drug in the body to achieve and then maintain the desired drug concentration. Consistent, prolonged delivery of medicine results in better patient compliance because patients do not require multiple administrations. Controlled-release formulations are also safer, as there is decrease of side effects related to dangerous spikes in drug concentrations. There is thus necessity for a CR formulation with lesser dosing frequency, preferably a single dose per day. In particular, there is a need in the field of art for an oral formulation of Drotaverine that provides controlled release of the drug while ensuring rapid onset of action. The present invention provides CR formulations of Drotaverine or salt thereof that release the drug from a single ingestible tablet or capsule so as to release the drug throughout the course of a day.

One of the embodiments of developed formulations of current invention exhibits 'Biphasic release profile' with the successful incorporation of 'instant and prolonged 'release portions for immediate and sustained pain relief.

### References:

1. Bolaji OO, Onyeji CO, Ogundaini AO, Olugbade TA, Ogunbona FA. Pharmacokinetics and bioavailability of Drotaverine in humans. Eur J Drug Metab Pharmacokinet. 1996; Jul-Sep; 21(3):217-21.
2. Blasko G. Pharmacology, mechanism of action and clinical significance of a convenient antispasmodic agent: Drotaverine. J Am Med Assoc Ind. Physicians' Update 1998; 1:63-9.
3. Om Prakash, S Saraf, M Rahman, Neeraj Agnihotri, and Vinay Pathak. Formulation and Evaluation of Floating Drotaverine Hydrochloride Tablets Using Factorial Design. Res J Pharm, Bio and Chem Sci, 2003; Oct-Dec; 4(4): 546-555.
4. Zhu, J. & Cao, Y. & Yu, M.-L & Liu, C. & He, W. & Zeng, B. & Li, J. Efficacy and safety of combination therapy with Drotaverine and ketorolac versus ketorolac monotherapy for acute renal colic: A retrospective study of 322 patients. International Journal of Clinical and Experimental Medicine. 2017; 10; 3454-3461.

In an embodiment, the present invention provides a formulation according to the appended claims.

In another embodiment, the present invention provides methods of preparation of controlled-release formulations of Drotaverine or salt thereof or similar active agents (those are prone to oxidative and hydrolytic degradation).

In another embodiment the present invention provides a formulation for use in the treatment of symptoms of gastrointestinal, biliary, urological and/or gynecological disorders characterized by spastic conditions of smooth muscles, by administering the controlled release formulations of the present invention.

Thus, in an important embodiment, the present invention provides stable CR formulation(s) comprising Drotaverine or salts thereof as a once or twice a day dosage using acidifying agents and polymers. The selected polymers have different hydrophilicity, hydrophobicity, swelling, erosion and pH dependent solubility characteristics which ensure flexibility in the control of drug release. The developed CR formulations can be administered as a multi-layered, multicoated tablet or capsule form. In some embodiments, the Drotaverine CR formulations disclosed herein can be dosed once a day leading to better patient convenience and compliance. They can be formulated as a single layer, bilayer or trilayer tablets or multicoated mini tablets or beads or pellets compressed as MUPS (Multiple-Unit Pellet System) tablets or filled in capsules. The capsule shells used can be of gelatin or non-gelatin based materials.

In order to identify the various parameters for a tablet formulation, trials of IR layer and CR (controlled release) layer were undertaken. The best prototype formulation having both IR and CR layer was selected on the basis of their dissolution profiles. Various concentrations of super disintegrants viz. Sodium Starch Glycollate, Croscarmellose Sodium, and Crospovidone were used alone or in combination to improve the IR- layer drug release and different grades of polymers alone or in combination at different concentrations are used to develop the CR-layer.

Oral ingestion is the most convenient and commonly employed route of drug delivery due to its ease of administration, high patient compliance, cost effectiveness, least sterility constraints, and flexibility in the design of the dosage form. Drotaverine has a fast onset of action that initiates relief from pain within 12 minutes after oral administration. Thus, an advantage of the compositions and methods of the present invention is to improve disease management by modifying the drug release rate profiles of Drotaverine drug delivery in comparison to IR tablets when administered orally. This will in turn lead to reduction of a dosing frequency from three times a day to once or twice a day, which provides greater convenience and better patient compliance.

In another embodiment, Drotaverine CR formulations of the present invention ensure uniform and therapeutically effective predictable plasma concentration level in blood stream for a period of 12-24 hours, thereby avoiding fluctuations of drug plasma level that are normally observed in IR tablet drug therapy. A once a day controlled release formulation also reduces the pill burden to patients. The CR formulations of Drotaverine or salt thereof provided by the present invention are designed in such a way that sufficient amount of the drug is released immediately within the 1^{st} hour to achieve plasma levels similar to the immediate release dosage form, and such that the rest of the drug is gradually released over a period of 24 hours to maintain the drug concentration within the therapeutic window. Thus, the formulations of the present invention assure a rapid and prolonged release of Drotaverine or salt thereof. Drotaverine CR formulations of the present invention avoid fluctuations of plasma levels with reduced side effects due to a simplified dosage schedule, compared with those of immediate-release dosage form, thereby improving patient compliance.

### SUMMARY OF THE INVENTION

The present invention relates to CR formulations of Drotaverine or salts thereof or similar antispasmodic agent. the formulations of present invention comprise Drotaverine or salts thereof, one or more polymers and one or more pharmaceutically acceptable excipients such that when administered orally the formulations release Drotaverine or salts thereof in a controlled release (CR) manner over a prolonged period of time (e.g., 12-24 hours). The term "*Controlled release*" used throughout the specification shall apply to dosage forms, matrices, particles, coatings, portions thereof, or compositions that alter the release of an active ingredient in any manner. Types of controlled release include modified, prolonged, sustained, extended, delayed, and the like.

In some aspects, the present invention relates to once or twice a day controlled release formulations of Drotaverine or salt thereof which avoid fluctuations of plasma levels, reduce pill burden and side effects, and which facilitate a simplified dosage schedule, thereby improving patient compliance. In some embodiments, the formulations release a sufficient amount of Drotaverine or salt thereof immediately within the 1^{st} hour after administration to achieve plasma levels similar to a conventional IR dosage form, thereby rapidly achieving minimal effective concentration (MEC). Thereafter, the drug is gradually released over a period of 12-24 hours to maintain the drug concentration within the therapeutic window viz. MEC.

In some embodiments, the CR formulations of the present invention employ polymers viz., Hypromellose, Hydroxyethyl Cellulose, Hydroxypropyl Cellulose, methyl cellulose, Carboxymethyl Cellulose or salts thereof, Ethyl Cellulose, Carbomers, Methacrylic acids and Polyethylene Oxides (non-ionic polymers) alone or in combination, acrylic copolymers and other tableting excipients and optionally one or more polymers from above list for film coating of compressed tablets.

In some embodiments, the CR formulations of the present invention also contain acidifying agents. The acidifying agent can be selected from, for example, Citric acid, Fumaric acid, Lactic acid, Maleic acid, Malic acid and Tartaric acid, alone or in combination.

The CR formulations may comprise other pharmaceutically acceptable excipients including fillers, flow aids, lubricants. Additional ingredients in the developed formulations may also include stabilizers e.g., acidifying agents, antioxidants etc.

Additionally, Drotaverine HCl or a similar type of drug molecule that is sensitive to oxygen may also require encapsulation or special coating or a microenvironment with inert gas e.g., nitrogen etc. in the finished product primary packing to further improve shelf-life. Suitable examples of drugs that are prone to oxidative degradation are selected from but not limited to Drotaverine, Mebeverine, Alevarine, Papaverine and pharmaceutically acceptable salts thereof.

Controlled release (CR) formulations deliver drug to body so as to establish therapeutically effective blood level of the active ingredient and once the blood level is achieved they continue to maintain constant blood levels for long durations by delivering the drug to body at the same rate as the body eliminates the drug. By avoiding peaks and troughs of the active ingredient in blood level associated with immediate release dosage forms, controlled drug delivery systems lower the incidence of adverse effects or side effects. Also, the controlled drug delivery systems reduce the frequency of dosing, leading to convenience to the patient in terms of dosing and compliance to the specified dosage regimens.

The present invention also describes CR dosage forms for Drotaverine, which has a fast (immediate) release fraction, which allows for sufficient concentration of the drug, Drotaverine, in the blood stream to produce quick therapeutic effect, and a slow (controlled) release fraction which maintains that therapeutic effect. Thus, in some embodiments, the present invention provides a controlled release dosage form of Drotaverine or salt thereof such that a sufficient amount of drug is released within about 1 hour after administration to achieve minimal effective concentration (MEC) levels similar to immediate release dosage form and such that the rest of the drug is released over a period of about 12 to 24 hours to achieve therapeutic efficacy as a once or twice a day drug delivery dosage form.

Further, in some aspects, the invention provides methods of preparation of controlled-release formulations of Drotaverine or a salt thereof. Also, in some embodiments, the invention further provides controlled-release formulations of Drotaverine or salt thereof for treating at least one symptom of gastrointestinal, biliary, urological and/or gynecological disorders characterized by spastic conditions of smooth muscles in a subject.
The aspects and embodiments of the present invention are provided herein:
An aspect of the present invention discloses a formulation comprising a controlled release portion and an immediate release portion in accordance with claim 1.

In an embodiment the present invention discloses that the formulation comprises 10 to 300 mg of Drotaverine or salt thereof.

The formulation may comprise Drotaverine or salt thereof in the range of 15% to 60% (w/w) of the formulation.

In still another embodiment the present invention discloses that the Drotaverine salt in the formulation is Drotaverine hydrochloride.

In still another embodiment the present invention discloses that at least one acidifying agent in the formulation is selected from the group consisting of Citric acid, Fumaric acid, Lactic acid, Maleic acid, Malic acid, Tartaric acid, and a combination thereof.

A further embodiment of the present invention discloses the polymer or mixture of polymers is selected from the group consisting of Hypromellose, Hydroxyethyl Cellulose, Hydroxypropyl Cellulose, hydrox-ypropyl methylcellulose, carboxymethyl cellulose, methyl cellulose, sodium carboxymethyl cel-lulose or salts thereof, Ethyl Cellulose, Carbomers, Methacrylic acids, Polyethylene Oxides (PEO), and a combination thereof.

The polymer may be Polyethylene oxide (PEO).

In still another embodiment the present invention discloses that the polymer or mixture of polymers is hydroxypropyl methylcellulose having an apparent viscosity ranging from 100-150,000 cP, wherein, optionally, the polymer or mixture of polymers is K100, K4M, K15M, K100M, E4M, E10M, Methocel K100M CR, or a combination thereof.

In yet another embodiment the present invention discloses that the formulation comprises controlled release polymer in the range of 5% to 30% (w/w) of the formulation.

In still another embodiment the present invention discloses that the at least one pharmaceutically acceptable excipient in the formulation is selected from the group consisting of gums, fillers, flow aids, lubricants, disintegrants, diluents, binders, lubricants, glidants, and a combination thereof.

In the formulation, the gums may be selected from the group consisting of xanthan gum, karaya gum, locust bean gum, alginic acid, sodium alginate, acrylic polymers, and a combination thereof; the diluents are selected from the group consisting of microcrystalline cellulose, lactose, dicalcium phosphate, starch, and a combination thereof; the binders are selected from the group consisting of starch, polyvinylpyrrolidone, natural or synthetic gum, cellulosic polymers, and a combination thereof; the lubricants and glidants are selected from the group consisting of talc, colloidal silicon dioxide, magnesium stearate, and a combination thereof; and the disintegrants are selected from the group consisting of Sodium Starch Glycollate, Croscar-mellose Sodium, Crospovidone, and a combination thereof.

The formulation may be for oral delivery.

The formulation may be a tablet, mini-tablet, MUPS (Multiple-Unit Pellet System) tablet or capsule.

The formulation may be in the form of single layered, bi-layered, multi-layered, coated, uncoated, or multi-coated pellets, granulates, or beads, which can be filled into capsules or compressed to tablets.

In a further embodiment the present invention discloses that the formulation optionally comprises a functional or non-functional coating.

The tablet or capsule may be prepared by a wet granulation, dry granulation/slugging or direct compression process.

The capsule may comprise shells prepared by gelatin or non-gelatin based materials.

The formulation may release Drotaverine or salt thereof for at least 24 hours.

The formulation may elicit a minimum effective concentration in plasma of a subject similar to conventional immediate release dosage form within about 1 hour and the rest of the drug is gradually released over a period of about 12-24 hours to maintain the drug concentration within the therapeutic window of Drotaverine.

In yet another embodiment the present invention discloses that the formulation has a dissolution release profile in-vitro of 25% to 40% after 1 hour, 30% to 50% after 2 hours, 40% to 65% after 4 hours, 60% to 85% after 8 hours and not less than 85% after about 16 hours.

Another aspect of the present invention discloses a method of preparing a single-layer controlled release tablet comprising Drotaverine or a salt thereof, the method comprising:
(a) sieving and dry mixing Drotaverine or salt thereof with an acidifying agent, polymer and excipient to obtain a drug-excipient blend;
(b) sieving and mixing extra-granular ingredients with the drug-excipient blend to obtain a formulation;
(c) compressing the formulation to form the tablet.

Another aspect of the present invention discloses a method of preparing a single or multi-layer tablet comprising Drotaverine or a salt thereof, the method comprising:
(a) sieving and dry mixing Drotaverine or salt thereof with an acidifying agent, polymer and excipient to obtain a dry mix;
(b) granulating the dry mix with a binder solution comprising at least polyvinylpyrrolidone and isopropyl alcohol to obtain granules;
(c) drying the granules to obtain a desired Loss-on Drying of the dried granules;
(d) milling the dried granules followed by sieving to obtain granules of specific size;
(e) sieving extra-granular ingredients followed by mixing with the granules of specific size to obtain a formulation; and
(f) compressing the formulation to form the tablet.

The method of preparing a single or multi-layer tablet comprising Drotaverine or a salt thereof may comprise coating the tablet with one or more functional or non-functional coatings.

An embodiment of the present invention discloses a use a formulation of the present invention for treating at least one symptom of a gastrointestinal, biliary, urological or gynecological disorder characterized by spastic conditions of smooth muscles in a subject, comprising administering the formulation to the subject.

### BRIEF DESCRIPTION OF THE DRAWINGS

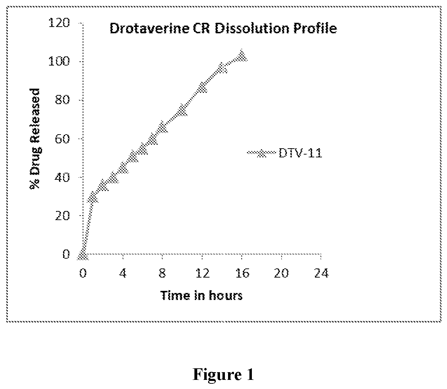

Figure 1: The figure shows a representative release rate profile of a CR formulation of Drotaverine hydrochloride prepared in accordance with the present invention. The dissolution profile is shown as % drug release of Drotaverine hydrochloride against time (in hours).

### DETAILED DESCRIPTION OF THE INVENTION

A large portfolio of polymers is available for CR drug delivery formulations/systems along with application expertise to meet specific formulation drug delivery which help development of new products. The controlled release polymer may be one or more Hypromellose, Hydroxyethyl Cellulose, Hydroxypropyl Cellulose, Carboxymethyl Cellulose or salts thereof, Ethyl Cellulose, Carbomers, Methacrylic acids and Polyethylene Oxides (nonionic polymers) are available in different pharmaceutical grades and offer a range of CR properties for a variety of dosage forms and processing methods. Variations in molecular weights and chemical substitutions provide innumerable ways to optimize formulation performance. Each range has fundamentally different hydrophilicity, hydrophobicity, wettability, pH dependent solubility, swelling and erosion characteristics to provide flexibility in control of the main mechanisms of release.

In some embodiments, the CR formulations of the present invention contain acidifying agents. The acidifying agent can be selected from, for example, Citric acid, Fumaric acid, Lactic acid, Maleic acid, Malic acid and Tartaric acid, or combinations thereof.

In some embodiments, the CR formulations of the present invention include Drotaverine or salts thereof, one or more hydrophilic polymers and one or more pharmaceutically acceptable excipients such that when administered orally the formulations release Drotaverine in a CR manner over a prolonged period of time.

In some embodiments, the formulations disclosed herein show an *in vitro* dissolution profile of Drotaverine or salts thereof, when measured using USP II Method, at 75 rpm, in 1000 ml, 0.1 N HCl (pH 1.2), at 37±0.5° C, to be between 25% and 40% released after 1 hour, between 30% and 50% released after 2 hours, between 40% and 65% released after 4 hours, and between 60% and 85% released after 8 hours and NLT (not less than) 85% after 16 hours. In some embodiments, the formulation releases a sufficient amount of Drotaverine drug which allows for sufficient concentration of the drug in the blood stream to produce a quick therapeutic effect and thereafter provides uniform and slow drug release for about 16 hours which then maintains that effect for about 24 hours duration. Hence, in some embodiments, the present invention provides a modified release dosage form which would release about 20% of the drug within about 1 hour and the balance of about 80% would be released thereafter to ensure therapeutic action for about 24 hours as a once or twice a day drug delivery dosage form.

The oral controlled release dosage formulations may include polymers of low molecular weight polyethylene oxide (PEO) alone or in combination with hydroxypropylmethylcellulose (HPMC) with a non-ionic polymer and other tableting excipients and optionally one or more enteric polymers.

Thus, the CR formulations of the present invention may comprise the following:
- Drotaverine or its salts thereof, in the range of between 10 to 300 mg, e.g., about 40 to about 240 mg. Drotaverine is preferably present in an amount suitable for single or twice a day dosing.
- One or more active ingredients which are similar types of drug molecules in that they are sensitive to oxidative degradation. Non-limiting examples of drugs that are prone to oxidative degradation include Drotaverine, Mebeverine, Alevarine, Papaverine and pharmaceutically acceptable salts thereof.
- Disintegrants/Superdisintegrants viz. Sodium Starch Glycollate, Croscarmellose Sodium, and Crospovidone, may be selected alone or in combination to improve the IR- layer drug release.
- Polyethylene oxide (PEO) as a non-ionic homopolymer of ethylene oxide (CAS Registry Number 25322-68-3). PEO is chemically similar to polyethylene glycol, but has a high molecular weight in the range of 100,000 to 7 million g/mol. Pharmaceutical grades of PEO may have unique properties such as non-ionic, highly swelling, hydrophilic and thermoplastic behaviour. PEOs are safe, non-toxic polymers that are not absorbed through the gastro-intestinal tract. PEO has an IID (Inactive Ingredient Database) limit of about 543.9 mg per tablet for oral applications.
- The "*hydrophilic controlled-release polymer*" may be selected, for instance, from one or more of cellulose derivatives selected from hydroxypropyl methylcellulose, hydroxyethyl cellulose, hydroxypropyl cellulose, carboxymethyl cellulose, methyl cellulose, sodium carboxymethyl cellulose; and gums selected from xanthan gum, karaya gum, locust bean gum, alginic acid and sodium alginate and acrylic polymers. The hydroxypropyl methylcellulose may be, for example, the commercially available products such as Methocel^{®} premium product grades having specific apparent viscosities, e.g., viscosities ranging from 100-150,000 cP (2% in water at 20° C) such as K100, K4M, K15M, K100M, E4M, E10M; viscosities ranging from 80000-120,000 cP (2% in water at 20° C) such as Methocel^{®} K100M CR. It was observed that the amount of the hydrophilic polymer per unit dose of Drotaverine or salt thereof plays a major role in the release characteristic of the formulation. The amount of hydrophilic controlled-release polymer may range from about 05-30 %w/w per unit weight of dosage form.
- The formulations may contain other release-retarding agents including hydrophobic polymers along with the combination of one or more hydrophilic polymers. However, hydrophilic polymers alone also can be used to obtain the controlled -release formulations with desirable characteristics achieved by the formulations disclosed herein.
- The controlled release formulation may also contain "*pharmaceutically acceptable excipients*" selected from, for example, one or more of diluents, binders, lubricants and glidants.
- The diluent may, for example, be selected from, for example, one or more of microcrystalline cellulose, lactose, dicalcium phosphate and starch.
- The binder may be selected from, for example, one or more of starch, polyvinylpyrrolidone, natural or synthetic gum and cellulosic polymers.
- The lubricants and glidants may be selected from, for example, one or more of talc, colloidal silicon dioxide and magnesium stearate.
- To protect Drotaverine hydrochloride from oxidative degradation, the developed formulations contain acidifying agents.
- The acidifying agent can be selected from, for example, alone or combination of Citric acid, Fumaric acid, Lactic acid, Maleic acid, Malic acid and Tartaric acid.
- The antioxidant can be selected from, for example, alone or combination of butylated hydroxyanisole (BHA), butylated hydroxytoluene (BHT), sodium metabisulfite, sodium thiosulfate, propyl gallate, ascorbic acid and cysteine.

The CR formulations of Drotaverine or salt thereof may be obtained in form of tablet, bead, pellet or capsule. The tablet may be an uncoated tablet, coated tablet, or mini-tablets. For instance, the CR formulations may be a single layer matrix tablet or bi-layer, tri-layer or multi-layered tablets with or without a functional or non-functional coating. A functional film coating is a coating that has a direct influence on the drug release of API (active pharmaceutical ingredient) of the solid oral dosage form (e.g. tablet, capsule, granule or pellet) examples include Ethyl cellulose dispersion with soluble polymer or enteric polymer based dispersion with water soluble ingredients. While a non-functional film coating is not directly influence the drug release of the API. The examples include HPMC based film costing dispersion with or without flavour to enhance the product acceptability of bitter tasting drugs like Drotaverine. The tablet may be prepared by wet granulation, dry granulation/slugging, or direct compression processes.

The input raw materials are selected to ensure there is minimal level of free water, which can be detrimental in causing degradation of Drotaverine hydrochloride. The manufacturing process, especially wet granulation process, required studies may be performed to adjust the levels of residual moisture content in the final drug product.

In some embodiments, the compressed tablets may be coated with suitable film forming compositions.

Based on the properties of Drotaverine IR tablets known in the field of art, the theoretical but hitherto unmet drug release pattern depicting bioavailability and pharmacokinetic properties for a 'once-a-day dosage form' of Drotaverine would be as follows:

| Time (hours) | % Release (Range) |
|---|---|
| 1 | 25-40 |
| 2 | 30-50 |
| 4 | 40-65 |
| 8 | 60-85 |
| 16 | NLT 85 |

The inventors of the present invention undertook several formulation trials comprising of one or combination of CR polymer, filler and other pharmaceutical auxiliary components and tried dry and wet granulation manufacturing process to arrive at the CR release formulations of Drotaverine or salt thereof described above. Initial experiments were undertaken as a monolayer and bi-layered tablet formulations. However, despite executing several trials, it was really difficult to control release profile as per theoretical release pattern which is evident in the following Tables 1 and 2 for monolayer tablets and Tables 3 and 4 for bi-layer tablets.

**Table 1: Mono-layer tablet formulations prepared by different manufacturing processes**

| Batch No. | DTV-01 | DTV-02 | DTV-03 | DTV-04 | DTV-05 | DTV-09 |
|---|---|---|---|---|---|---|
| Mfg . Process | Slugging (DG) | Slugging (DG) | Slugging (DG) | Wet gran. (IPA) | Wet gran. (IPA) | Wet gran. (IPA) |
| Material | Percent (%) | | | | | |
| Drotaverine Hydrochloride | 26.7 | 26.7 | 26.7 | 26.7 | 26.7 | 53.3 |
| Polyox WSR 303 | | 50 | 45 | | 15 | |
| Methocel HPMC K 15 M | - | - | - | | 15 | 10 |
| Polyox WSR 301 | | | | 20 | | 14.9 |
| MCC | 45 | - | 10 | 23.3 | 18.3 | 11.1 |
| Methocel HPMC K 100 M | 27.3 | 22.3 | 17.3 | 20 | 15 | |
| PVP K-30 | | | | 4 | 4 | 6 |
| IPA | | | | | | |
| MCC | | | | 5 | 5 | 3.3 |
| Colloidal Silicon dioxide | 0.5 | 0.5 | 0.5 | 0.5 | 0.5 | 0.7 |
| Lubrication | | | | | | |
| Magnesium Stearate | 0.5 | 0.5 | 0.5 | 0.5 | 0.5 | 0.7 |

**Table-2: Dissolution Release profile of mono-layer tablet formulations of Table 1**

| Dissolution Release profile | | | | | | |
|---|---|---|---|---|---|---|
| Time (Target Release) | % | % | % | % | % | % |
| Batch No. | DTV-01 | DTV-02 | DTV-03 | DTV-04 | DTV-05 | DTV-09 |
| 1 hour (25-40%) | 7 | 5 | 6 | 6 | 6 | 10 |
| 2 hours (30-50%) | 12 | 8 | 9 | 11 | 10 | 18 |
| 4 hours (40-65%) | 26 | 18 | 16 | 23 | 19 | 38 |
| 8 hours (60-85%) | 61 | 40 | 40 | 54 | 37 | 73 |
| 16 hours (NLT 85%) | 100 | 100 | 100 | 100 | 100 | 100 |

**Table-3: Bi-layer tablets formulations**

| Component | Batch No. (% w/w) | | | | |
|---|---|---|---|---|---|
| | DTV-15 | DTV-42 | DTV-45 | DTV-56 | DTV-57 |
| Drug | 36 | 40 | 40 | 40 | 40 |
| CR Polymer | 45 | 36 | 22.5 | 13 | 29 |
| Filler | 14 | 15 | 26 | 37 | 21 |
| Binder | 4.7 | 5 | 6 | 5 | 4 |
| Flow aid | 0.5 | 1 | 1 | 1 | 1 |
| Disintegrant | - | 1 | 1.5 | 3 | 3 |
| Stabilizer | - | 2 | 3 | 1 | 2 |
| Colorant | 0.02 | 0.02 | 0.02 | 0.02 | 0.02 |

**Table-4: Dissolution Release profile of bi-layer tablet formulations of Table 2**

| Time (hours) | Target release Range (%) | (%) | (%) | (%) | (%) | (%) |
|---|---|---|---|---|---|---|
| Batch No. | | DTV-15 | DTV-42 | DTV-45 | DTV-56 | DTV-57 |
| 1 | 25-40 | 36 | 24 | 25 | 20 | 20 |
| 2 | 30-50 | 45 | 40 | 39 | 33 | 32 |
| 4 | 40-65 | 59 | 41 | 47 | 45 | 41 |
| 8 | 60-85 | 93 | 51 | 53 | 63 | 59 |
| 16 | NLT 85% | 100 | 66 | 70 | 86 | 84 |

Subsequently, few of these trial batch tablets were exposed to accelerated stability conditions and evaluated for impurity profiling. For understanding chemical degradation characteristics of the CR formulations, a prototype formulation was exposed to 'forced degradation studies' and results are presented in below Table 5. It was observed that there were unknown impurities at levels of nearly 2-3% w/w for accelerated condition exposed samples. Therefore, in order to develop stable CR formulations with desired profile, pre-formulation studies of Drotaverine drug characteristics were undertaken to determine some of key physicochemical properties such as solubility, dissolution, assay and related substances parameters and drug stability characteristics etc.

**Table 5: Chemical degradation characteristics of proposed CR formulation exposed to 'Forced/accelerated degradation studies'**

| Batch No. | DTV-042 | | DTV-042 | | DTV-042 | | DTV-042 | |
|---|---|---|---|---|---|---|---|---|
| Condition | CONTROL | | ACID Degradation | | BASE Degradation | | PEROXIDE Degradation | |
| Name of Impurity | RRT | % w/w | RRT | % w/w | RRT | % w/w | RRT | % w/w |
| Drotaverine Amine | No impurities observed | | | | | | | |
| Drotaverine Acid | | | | | | | | |
| Drotaverine Amide | | | | | | | | |
| Drotaverladine | 1.67 | 0.02 | 1.67 | 0.03 | 1.67 | 1.08 | 1.66 | 0.91 |
| Highest individual unknown impurity | | 0.20 | | 0.20 | | 0.20 | | 3.15 |
| Total Impurities | | 0.51 | | 0.76 | | 1.89 | | 24.58 |

The results of the study were most surprising. In view of the fact that Drotaverine is susceptible to oxidative and hydrolytic degradation, it was assumed that adding an antioxidant would increase the stability thereof. However, addition of an antioxidant improved the stability only marginally. On the contrary, and to the utmost surprise of the inventors, addition of an acidifying agent resulted in remarkable improvement in stability resulting in a controlled release formulation of Drotaverine. The study demonstrates that incorporation of small amount of antioxidant to obtain stable CR release formulations is advantageous in order to avoid oxidative degradation of Drotaverine or salt thereof or active agents which are prone to oxidative/hydrolytic degradation. Additionally, the results indicate that addition of an acidifier alone or in combination with minor amounts of antioxidant also helps in developing stable CR formulation of Drotaverine and salt thereof.

The inventors of present invention surprisingly found that stable CR dosage formulations of Drotaverine or salt thereof with reduced degradation (and having 12-24 hours duration of release profile) can be achieved only with addition of acidifying agent and without or with minor amounts of antioxidant. The inventors further developed different CR formulations as monolayer or multilayer; MUPS tablets, coated, uncoated, tablets, as pellets or mini-tablets filled in capsules. These developed tablets may be coated with non-functional or functional coating. The capsule shells are based on gelatin or non-gelatin polymer. The formulations of the present invention can be tablets or capsules containing immediate and prolonged release portions demonstrating biphasic drug release profile. This ensures instant bioavailability similar to IR tablets, which is particularly desirable in the case of patients suffering from pain conditions. The IR portion comprises a mixture of excipients and stabilizers and suitable quantity of active substance allowing immediate release action similar to IR tablets along with CR drug portion comprising appropriate quantities of polymers, excipients and stabilizers interspersed with suitable portion of active drug ensuring medicament release in a controlled manner over 12-24 hours duration.

The present invention provides a controlled-release formulation comprising Drotaverine or salt thereof, at least one acidifying agent and a polymer or mixture of polymers along with at pharmaceutically acceptable excipients wherein said formulation is substantially free of anti-oxidants. The anti-oxidant is present from 0 to 10% (w/w) of the formulation.

Exemplary CR formulations of the present invention are provided in Table 6 with % range of respective ingredients as per developed formulations.

**Table 6: Exemplary CR formulations of the present invention**

| Component | % w/w range |
|---|---|
| Active Drug | 15-60 |
| Filler | 13-55 |
| Colorant | 0.1-0.6 |
| Stabilizer | 0.5-10 |
| Binder | 3-9 |
| Disintegrant | 0.5-8 |
| Flow aid | 0.2-1.5 |
| Lubricant | 0.2-2 |
| CR Polymer | 5-30 |

The developed CR formulations were prepared in different dosage forms viz. tablets or capsules prepared by direct blending, direct compression, dry granulation or wet granulation-based technology. For wet granulation manufacturing process, the granulating fluid may be hydro-alcoholic or non-aqueous granulating fluid viz. alcoholic solvents.

The method of preparing a single-layer controlled release tablet comprising formulation of Drotaverine or salt thereof comprises (a) sieving and dry mixing active ingredients with acidifying agent, polymer and excipient to obtain a drug-excipient blend; (b) sieving and mixing extra-granular ingredients with the drug-excipient blend obtained in step (a); (c) compressing the formulation of step (b) to form the tablet.

The method of preparing single or multi-layered tablet comprising Drotaverine or salt thereof, the method comprising: (a) sieving and dry mixing active ingredients with acidifying agent, polymer and excipient to obtain dry mix;(b) granulating dry mix by binder solution comprising at least polyvinylpyrrolidone and isopropyl alcohol to obtain granules; (c) drying granules to obtain desired Loss-on Drying of dried granules; (d) milling dried granules followed by sieving to obtain granules of specific size; (e) sieving extra-granular ingredients followed by mixing with granules obtained in step 'd'; (f) compressing the formulation of step (e) to form the tablet.

The following non-limiting examples further illustrate the CR formulations of Drotaverine or salt thereof, and process of making such formulations.

### REFERENCE EXAMPLE 1:

Drotaverine CR formulations prepared as '*Single Layer*' tablets by Direct Compression method in following manner:

| **Ingredient** | |
|---|---|
| | **%w/w** |
| **Dry mixing** | |
| Drotavarine HCl | 26.7 |
| Non-ionic poly (ethylene oxide) polymer (Polyox WSR 301) | 10 |
| Water-soluble high molecular weight poly(ethylene oxide) polymer (Polyox WSR 303) | 45 |
| MCC | 17.3 |

| **Extra-granular** | |
|---|---|
| Colloidal silicon dioxide | 0.5 |
| Magnesium stearate | 0.5 |

### REFERENCE EXAMPLE 2:

Drotaverine CR formulation prepared as '*Single Layer*' tablets by Wet Granulation method in the following manner:

| **Ingredient** | |
|---|---|
| | **%w/w** |
| **Dry mixing** | |
| Drotavarine HCl | 26.67 |
| HPMC K 15 | 15 |
| HPMC K 100 | 10 |
| Polyox WSR 303 | 20 |
| MCC | 18.3 |

| **Binder Preparation** | |
|---|---|
| IPA | q.s. |
| PVP K 30 | 4 |

| **Extra-granular** | |
|---|---|
| MCC | 5 |
| Colloidal silicon dioxide | 0.5 |
| Magnesium stearate | 0.5 |

### EXAMPLE 3:

Drotaverine CR formulation prepared as *'Bi-Layer'* tablets by Wet Granulation method:

| **Ingredient** | **Bi-layered** | |
|---|---|---|
| | **IR portion** | **CR portion** |
| | **%w/w** | **%w/w** |
| **Dry mixing** | | |
| Drotavarine HCl | 54.55 | 52.94 |
| HPMC K 100 | - | 17.65 |
| Polyox WSR 301 | - | 17.65 |
| Sodium starch glycolate | 3.64 | - |
| MCC | 31.8 | 5.88 |
| Brilliant Blue | 0.2 | - |

| **Binder Preparation** | | |
|---|---|---|
| IPA | q.s. | q.s. |
| PVP K 30 | 4.5 | 3.53 |

| **Extra granular** | | |
|---|---|---|
| MCC | 4.5 | 1.47 |
| Colloidal silicon dioxide | 0.5 | 0.44 |
| Magnesium stearate | 0.5 | 0.44 |

### EXAMPLE 4:

Drotaverine CR formulation prepared as 'Bi-Layer' tablets by Wet Granulation method:

| **Ingredient** | **Bi-layered** | |
|---|---|---|
| | **IR portion** | **CR portion** |
| | **%w/w** | **%w/w** |
| **Dry mixing** | | |
| Drotavarine HCl | 40 | 40 |
| HPMC K 100 | - | 22.44 |
| Polyox WSR 303 | - | 22.44 |
| Sodium starch glycolate | 4.8 | - |
| MCC | 42 | 7.56 |
| Brilliant Blue | 0.2 | |

| **Binder Preparation** | | |
|---|---|---|
| IPA | q.s. | q.s. |
| PVP K 30 | 6 | 4.67 |

| **Extra-granular** | | |
|---|---|---|
| MCC | 6 | 2 |
| Colloidal silicon dioxide | 0.6 | 0.44 |
| Magnesium stearate | 0.6 | 0.44 |

### EXAMPLE 5:

Drotaverine CR formulation prepared as 'Bi-Layer' tablets by Wet Granulation method:

| **Ingredient** | **Bi-layered** | |
|---|---|---|
| | **IR portion** | **CR portion** |
| | **%w/w** | **%w/w** |
| **Dry mixing** | | |
| Drotavarine HCl | 46.67 | 37.78 |
| HPMC K 100 | - | 22.44 |
| Polyox WSR 303 | - | 22.44 |
| Sodium starch glycolate | 4.8 | - |
| MCC | 35.3 | 9.78 |
| Brilliant Blue | 0.2 | - |

| **Binder Preparation** | | |
|---|---|---|
| IPA | q.s. | q.s. |
| PVP K 30 | 6 | 4.67 |

| **Extra-granular** | | |
|---|---|---|
| MCC | 6 | 2 |
| Colloidal silicon dioxide | 0.6 | 0.44 |
| Magnesium stearate | 0.6 | 0.44 |

Optionally the compressed tablet can be coated with functional and non-functional coating agents.

### EXAMPLE 6:

Drotaverine CR formulation prepared as 'Bi-Layer' tablets by Wet Granulation method (60 mg strength)

| **Ingredient** | **Bi-layered** | |
|---|---|---|
| | **IR portion** | **CR portion** |
| | **mg** | **mg** |
| **Dry mixing** | | |
| Drotavarine HCl | 15 | 45 |
| HPMC K 100 | - | 25 |
| Polyox WSR 303 | - | 25 |
| Sodium starch glycolate | 2 | - |
| MCC | 16 | 9 |
| Brilliant Blue | 0 | |

| **Binder Preparation** | | |
|---|---|---|
| IPA | q. s. | q.s. |
| PVP K 30 | 2 | 5 |

| **Extra-granular** | | |
|---|---|---|
| MCC | 2 | 2 |
| Colloidal silicon dioxide | 0.2 | 0.5 |
| Magnesium stearate | 0.2 | 0.5 |

### EXAMPLE 7:

Drotaverine CR formulation prepared as 'Bi-Layer' tablets by Wet Granulation method (120 mg strength)

| **Ingredient** | **Bi-layered** | |
|---|---|---|
| | **IR portion** | **CR portion** |
| | **mg** | **mg** |
| **Dry mixing** | | |
| Drotavarine HCl | 30 | 90 |
| HPMC K 100 | - | 50 |
| Polyox WSR 303 | - | 50 |
| Sodium starch glycolate | 4 | - |
| MCC | 32 | 17 |
| Brilliant Blue | 0.2 | |

| **Binder Preparation** | | |
|---|---|---|
| IPA | q. s. | q.s. |
| PVP K 30 | 5 | 11 |

| **Extra-granular** | | |
|---|---|---|
| MCC | 5 | 5 |
| Colloidal silicon dioxide | 0.4 | 1 |
| Magnesium stearate | 0.4 | 1 |

### EXAMPLE 8:

Drotaverine CR formulation prepared as 'Bi-Layer' tablets by Wet Granulation method (180 mg strength)

| **Ingredient** | **Bi-layered** | |
|---|---|---|
| | **IR portion** | **CR portion** |
| | **mg** | **mg** |
| **Dry mixing** | | |
| Drotavarine HCl | 45 | 135 |
| HPMC K 100 | - | 76 |
| Polyox WSR 303 | - | 76 |
| Sodium starch glycolate | 5 | - |
| MCC | 47 | 26 |
| Brilliant Blue | 0 | |

| **Binder Preparation** | | |
|---|---|---|
| IPA | q.s. | q.s. |
| PVP K 30 | 7 | 16 |

| **Extra-granular** | | |
|---|---|---|
| MCC | 7 | 7 |
| Colloidal silicon dioxide | 1 | 2 |
| Magnesium stearate | 1 | 2 |

### EXAMPLE 9:

Drotaverine CR formulation prepared as 'Bi-Layer' tablets by Wet Granulation method (240 mg strength).

| **Ingredient** | **Bi-layered** | |
|---|---|---|
| | **IR portion** | **CR portion** |
| | **mg** | **mg** |
| **Dry mixing** | | |
| Drotavarine HCl | 60 | 180 |
| HPMC K 100 | - | 100.96 |
| Polyox WSR 303 | - | 100.96 |
| Sodium starch glycolate | 7.2 | - |
| MCC | 63.2 | 34 |
| Brilliant Blue | 0.32 | |

| **Binder Preparation** | | |
|---|---|---|
| IPA | q.s. | q.s. |
| PVP K 30 | 9 | 21 |

| **Extra-granular** | | |
|---|---|---|
| MCC | 9 | 9 |
| Colloidal silicon dioxide | 0.8 | 2 |
| Magnesium stearate | 0.8 | 2 |

### EXAMPLE 10:

Drotaverine CR formulation granules prepared by Wet Granulation method (240 mg strength)

| **Ingredients** | **IR Portion (in mg)** | **CR Portion (in mg)** |
|---|---|---|
| **Intra-granular Material** | | |
| Drotaverine HCl | 75.00 | 165.00 |
| Sodium Starch Glycollate | 7.00 | |
| MCC | 35.70 | 133.60 |
| HPMC K 100M | - | 54.00 |
| HPMC K 4M | - | 54.00 |
| Colloidal Silicon Dioxide | - | 2.00 |
| Colouring agent | 0.20 | - |

| **Binder Solution** | | |
|---|---|---|
| Citric Acid | 3.60 | 8.40 |
| IPA | q.s. | q.s. |
| PVP K 30 | 9.00 | 25.00 |

| **Extra-granular Material** | | |
|---|---|---|
| MCC | 18.00 | 5.00 |
| Colloidal Silicon Dioxide | 0.45 | 1.00 |
| Colouring agent | 0.15 | |
| Magnesium Stearate | 0.90 | 2.00 |

### EXAMPLE 11:

Drotaverine CR formulation granules prepared by Wet Granulation method (240 mg strength)

| **Ingredients** | **IR Portion (in mg)** | **ER Portion (in mg)** |
|---|---|---|
| **Intra-granular Material** | | |
| Drotaverine HCl | 60.00 | 180.00 |
| Sodium Starch Glycollate | 7.00 | - |
| MCC | 23.40 | 186.10 |
| HPMC K 100M | - | 38.40 |
| HPMC K 4M | - | 38.40 |
| Colloidal Silicon Dioxide | - | 2.00 |
| Colouring agent | 0.20 | - |
| Sodium Metabisulfite | 0.90 | 2.1 |

| **Binder Solution** | | |
|---|---|---|
| IPA | q.s. | q.s. |
| PVP K 30 | 9.00 | 25.00 |

| **Extra-granular Material** | | |
|---|---|---|
| MCC | 18.00 | 5.00 |
| Colloidal Silicon Dioxide | 0.45 | 1.00 |
| Colouring agent | 0.15 | - |
| Magnesium Stearate | 0.90 | 2.00 |

### EXAMPLE 12:

Drotaverine CR formulation granules prepared by Wet Granulation method (240 mg strength)

| **Ingredients** | **IR Portion (in mg)** | **ER Portion (in mg)** |
|---|---|---|
| **Intra granular Material** | | |
| Drotaverine HCl | 60.00 | 180.00 |
| Sodium Starch Glycollate | 7.20 | - |
| MCC | 60.80 | 29.50 |
| HPMC K 100M | - | 100.96 |
| Polox WSR 303 | - | 100.96 |
| Colloidal Silicon Dioxide | - | 2.00 |
| Colouring agent | 0.20 | - |
| Sodium Metabisulfite | 1.50 | 4.5 |

| **Binder Solution** | | |
|---|---|---|
| IPA | q.s. | q.s. |
| PVP K 30 | 9.00 | 21.00 |

| **Extra granular Material** | | |
|---|---|---|
| MCC | 9.45 | 7.08 |
| Colloidal Silicon Dioxide | 0.80 | 2.00 |
| Colouring agent | 0.15 | - |
| Magnesium Stearate | 0.90 | 2.00 |

### EXAMPLE 13:

Drotaverine CR formulation granules prepared by Wet Granulation method (240 mg strength)

| **Ingredients** | **IR Portion (in mg)** | **CR Portion (in mg)** |
|---|---|---|
| **Intra-granular Material** | | |
| Drotaverine HCl | 60.00 | 180.00 |
| Sodium Starch Glycollate | 7.00 | |
| MCC | 24.30 | 188.20 |
| HPMC K 100M | - | 38.40 |
| HPMC K 4M | - | 38.40 |
| Colloidal Silicon Dioxide | - | 2.00 |
| Colouring agent | 0.20 | - |

| **Binder Solution** | | |
|---|---|---|
| IPA | q.s. | q. s. |
| PVP K 30 | 9.00 | 25.00 |

| **Extra-granular Material** | | |
|---|---|---|
| MCC | 18.00 | 5.00 |
| Colloidal Silicon Dioxide | 0.45 | 1.00 |
| Colouring agent | 0.15 | |
| Magnesium Stearate | 0.90 | 2.00 |

### EXAMPLE 14:

Drotaverine CR formulation granules prepared by Wet Granulation method (240 mg strength)

| **Ingredients** | **IR Portion (in mg)** | **CR Portion (in mg)** |
|---|---|---|
| **Intra-granular Material** | | |
| Drotaverine HCl | 75.00 | 165.00 |
| Sodium Starch Glycollate | 7.00 | |
| MCC | 34.80 | 131.50 |
| HPMC K 100M | - | 54.00 |
| HPMC K 4M | - | 54.00 |
| Colloidal Silicon Dioxide | - | 2.00 |
| Colouring agent | 0.20 | - |
| Sodium Metabisulfite | 0.9 | 2.1 |

| **Binder Solution** | | |
|---|---|---|
| Citric Acid | 3.6 | 8.4 |
| IPA | q.s. | q.s. |
| PVP K 30 | 9.00 | 25.00 |

| **Extra-granular Material** | | |
|---|---|---|
| MCC PH | 18.00 | 5.00 |
| Colloidal Silicon Dioxide | 0.45 | 1.00 |
| Colouring agent | 0.15 | |
| Magnesium Stearate | 0.90 | 2.00 |

### EXAMPLE 15:

Drotaverine CR formulation granules prepared by Wet Granulation method (240 mg strength)

| **Ingredients** | **IR Portion (in mg)** | **CR Portion (in mg)** |
|---|---|---|
| **Intra granular Material** | | |
| Drotaverine HCl | 75.00 | 165.00 |
| Crospovidone | 10.00 | |
| MCC | 31.80 | 131.50 |
| HPMC K 100M | - | 80.00 |
| HPMC K 4M | - | 28.00 |
| Colloidal Silicon Dioxide | - | 2.00 |
| Colouring agent | 0.20 | - |
| Sodium Metabisulfite | 2.0 | 2.1 |

| **Binder Solution** | | |
|---|---|---|
| Maleic Acid | 2.5 | 8.4 |
| IPA | q. s. | q.s. |
| PVP K 30 | 15.00 | 25.00 |

| **Extra-granular Material** | | |
|---|---|---|
| MCC | 12.00 | 5.00 |
| Colloidal Silicon Dioxide | 0.45 | 1.00 |
| Colouring agent | 0.15 | |
| Magnesium Stearate | 0.90 | 2.00 |

### EXAMPLE 16:

Drotaverine CR formulation granules prepared by Wet Granulation method (240 mg strength)

| **Ingredients** | **IR Portion (in mg)** | **CR portion (in mg)** |
|---|---|---|
| **Intra granular Material** | | |
| Drotaverine HCl | 75.00 | 165.00 |
| Crospovidone | 10.00 | |
| MCC | 31.80 | 131.50 |
| HPMC K 100M | - | 50.00 |
| HPMC K 4M | - | 58.00 |
| Colloidal Silicon Dioxide | - | 2.00 |
| Colouring agent | 0.20 | - |
| Butyl hydroxy Toluene | 1.0 | 2.1 |

| **Binder Solution** | | |
|---|---|---|
| Tartaric acid | 3.5 | 8.4 |
| IPA | q.s. | q.s. |
| PVP K 30 | 15.00 | 25.00 |

| **Extra granular Material** | | |
|---|---|---|
| MCC | 12.00 | 5.00 |
| Colloidal Silicon Dioxide | 0.45 | 1.00 |
| Colouring agent | 0.15 | |
| Magnesium Stearate | 0.90 | 2.00 |

### EXAMPLE 17:

Drotaverine CR formulation granules prepared by Wet Granulation method (240 mg strength)

| **Ingredients** | **IR Portion (in mg)** | **CR portion (in mg)** |
|---|---|---|
| **Intra granular Material** | | |
| Drotaverine HCl | 75.00 | 165.00 |
| Crospovidone | 10.00 | |
| MCC | 31.80 | 131.50 |
| HPMC K 100M | - | 50.00 |
| HPMC K 4M | - | 58.00 |
| Colloidal Silicon Dioxide | - | 2.00 |
| Colouring agent | 0.20 | - |
| Butyl hydroxy Toluene | 1.0 | 2.1 |

| **Binder Solution** | | |
|---|---|---|
| Tartaric acid | 3.5 | 8.4 |
| IPA | q.s. | q.s. |
| PVP K 30 | 15.00 | 25.00 |

| **Extra granular Material** | | |
|---|---|---|
| MCC | 12.00 | 5.00 |
| Colloidal Silicon Dioxide | 0.45 | 1.00 |
| Colouring agent | 0.15 | |
| Magnesium Stearate | 0.90 | 2.00 |

### REFERENCE EXAMPLE 18:

Drotaverine CR formulation MUPS (Multiple-Unit Pellet System) tablets (240 mg strength)

| **MUPS Tablets** | |
|---|---|
| **Ingredients** | **Qty. Per unit (in mg)** |
| Drotaverine HCl | 240.00 |
| HPMC | 12.00 |
| Polysorbate -80 | 12.00 |
| Citric Acid | 6.00 |
| Talc | 5.00 |
| Sugar Spheres 355-425 µm | 80.00 |
| Ethyl Cellulose Coating dispersion | 20.00 |
| Microcrystalline Cellulose (MCC) | 173.00 |
| Magnesium Stearate | 4.00 |
| Colloidal Silicon Dioxide | 4.00 |

### REFERENCE EXAMPLE 19:

Drotaverine CR formulation capsules with CR pellets (240 mg strength)

| **Capsule with CR Pellets** | |
|---|---|
| **Ingredients** | **Qty. Per capsule (in mg)** |
| Drotaverine HCl | 240.00 |
| HPMC | 12.00 |
| Polysorbate -80 | 12.00 |

| | |
|---|---|
| Fumaric Acid | 5.00 |
| Butyl Hydroxy Anisole | 3.00 |
| Talc | 5.00 |
| Sugar Spheres 355-425 µm | 80.00 |
| Ethyl Cellulose Coating dispersion | 20.00 |
| Starch | 15.00 |
| Stearic Acid | 4.00 |
| Colloidal Silicon Dioxide | 4.00 |

### In-vitro dissolution profile

The formulations of the present invention are illustrated in but not limited to the examples given hereinabove. A representative drug dissolution release rate profile for an exemplary formulation of the present invention is depicted in Figure 1. The developed formulation is evaluated for '*In-vitro* dissolution profile' using USP II Method, at 75 rpm, in 1000 ml, 0.1 N HCl (pH 1.2), at 37±0.5° C. The dissolution release profile of CR formulations of the present invention *in-vitro* is found to be between about 25% and about 40% released after about 1 hour, between about 30% and about 50% released after about 2 hours, between about 40% and about 65% released after about 4 hours, and between about 60% and about 85% released after about 8 hours and NLT about 85% after about 16 hours.

### Chemical Stability:

Impurities in new drug products such as degradation products of the drug substance or reaction products of the drug substance with an excipient and/or immediate container closure system are collectively referred to as "degradation products". Generally, impurities present in the new drug substance need not be monitored or specified in the new drug product unless they are also degradation products.

The present invention provides stable CR formulations of Drotaverine or salt thereof or similar active agents which are prone to oxidative / hydrolytic degradation. The developed formulations have improved chemical stability wherein individual unknown impurity levels are less than 0.2%w/w (as per currently available globally acceptable standards for drug products).

### Non-limiting advantages of the CR formulations of the present invention:

1. The CR formulations of Drotaverine, developed by extensive trials and developing effective ratios of polymers, binders, excipients vis-à-vis active drug, achieve a target drug release rate from a single ingestible dosage form and achieves maintenance of a target MEC for about 24 hours.
2. The CR formulations disclosed herein are designed to achieve an immediate onset of action and sustained release profile. For instance, a sufficient (therapeutic) amount of the Drotaverine drug is released within about 1 hour after administration to achieve plasma levels similar to an immediate release dosage form and the rest of the drug is gradually released over a period of about 12-24 hours to maintain the drug concentration within the therapeutic window.
3. The CR formulations of Drotaverine are capable of being formulated as single or multi-layered tablets or multicoated mini-tablets, pellets or beads filled in capsules.
4. The formulations of Drotaverine are capable of being manufactured by direct compression, dry granulation, wet granulation or fluidized bed processing methods.

## Claims

1. A formulation comprising a controlled release portion and an immediate release portion, wherein the formulation comprises Drotaverine or a salt thereof, a polymer or mixture of polymers, and at least one pharmaceutically acceptable excipient, wherein said formulation comprises at least one acidifying agent and from 0 to 10% by weight of anti-oxidants.

2. The formulation of claim 1, wherein the formulation comprises 10 to 300 mg of Drotaverine or salt thereof.

3. The formulation of claim 1 or claim 2, wherein the Drotaverine salt is Drotaverine hydrochloride.

4. The formulation of claim 1, wherein said at least one acidifying agent is selected from the group consisting of Citric acid, Fumaric acid, Lactic acid, Maleic acid, Malic acid, Tartaric acid, and a combination thereof.

5. The formulation of claim 1, wherein the polymer or mixture of polymers is selected from the group consisting of Hypromellose, Hydroxyethyl Cellulose, Hydroxypropyl Cellulose, hydroxypropyl methylcellulose, carboxymethyl cellulose, methyl cellulose, sodium carboxymethyl cellulose or salts thereof, Ethyl Cellulose, Carbomers, Methacrylic acids, Polyethylene Oxides (PEO), and a combination thereof.

6. The formulation of claim 5, wherein the polymer or mixture of polymers is hydroxypropyl methylcellulose having an apparent viscosity ranging from 0.1-150 Pa·s (100-150,000 cP) (2% in water at 20° C).

7. The formulation of any one of the preceding claims, wherein the formulation comprises controlled-release polymer in the range of 5% to 30% (w/w) of the formulation.

8. The formulation of claim 1, wherein the at least one pharmaceutically acceptable excipient is selected from the group consisting of gums, fillers, flow aids, lubricants, disintegrants, diluents, binders, lubricants, glidants, and a combination thereof.

9. The formulation of claim 1, comprising up to 10% by weight of anti-oxidant.

10. The formulation of claim 9, wherein the antioxidant is chosen from butylated hydroxyanisole (BHA), butylated hydroxytoluene (BHT), sodium metabisulfite, sodium thiosulfate, propyl gallate, ascorbic acid and cysteine, or combinations thereof.

11. The formulation of any of the preceding claims, wherein the formulation optionally comprises a functional or non-functional coating.

12. The formulation of any one of the preceding claims, wherein the formulation has a dissolution release profile *in vitro,* when measured using USP II Method, at 75 rpm, in 1000 ml, 0.1 N HCl (pH 1.2), at 37±0.5° C, of 25% to 40% after 1 hour, 30% to 50% after 2 hours, 40% to 65% after 4 hours, 60% to 85% after 8 hours and not less than about 85% after 16 hours.

13. A method of preparing a single-layer controlled release tablet comprising Drotaverine or a salt thereof, the method comprising:
(a) sieving and dry mixing Drotaverine or salt thereof with an acidifying agent, polymer and excipient to obtain a drug-excipient blend;
(b) sieving and mixing extra-granular ingredients with the drug-excipient blend to obtain a formulation;
(c) compressing the formulation to form the tablet.

14. A method of preparing a single or multi-layer tablet comprising Drotaverine or a salt thereof, the method comprising:
(a) sieving and dry mixing Drotaverine or salt thereof with an acidifying agent, polymer and excipient to obtain a dry mix;
(b) granulating the dry mix with a binder solution comprising at least polyvinylpyrrolidone and isopropyl alcohol to obtain granules;
(c) drying the granules to obtain a desired Loss-on Drying of the dried granules;
(d) milling the dried granules followed by sieving to obtain granules of specific size;
(e) sieving extra-granular ingredients followed by mixing with the granules of specific size to obtain a formulation; and
(f) compressing the formulation to form the tablet.

15. A formulation of any of claims 1 to 12 for use in the treatment of at least one symptom of a gastrointestinal, biliary, urological or gynecological disorder **characterized by** spastic conditions of smooth muscles in a subject, comprising administering the formulation to the subject.

## Patentansprüche

1. Formulierung, umfassend einen Teil mit kontrollierter Freisetzung und einen Teil mit sofortiger Freisetzung, wobei die Formulierung Drotaverin oder ein Salz davon, ein Polymer oder eine Mischung von Polymeren und mindestens einen pharmazeutisch unbedenklichen Hilfsstoff umfasst, wobei die Formulierung mindestens ein Ansäuerungsmittel und 0 bis 10 Gew.-% Antioxidantien umfasst.

2. Formulierung nach Anspruch 1, wobei die Formulierung 10 bis 300 mg Drotaverin oder Salz davon umfasst.

3. Formulierung nach Anspruch 1 oder Anspruch 2, wobei das Drotaverinsalz Drotaverinhydrochlorid ist.

4. Formulierung nach Anspruch 1, wobei das mindestens eine Ansäuerungsmittel ausgewählt ist aus der Gruppe bestehend aus Zitronensäure, Fumarsäure, Milchsäure, Maleinsäure, Äpfelsäure, Weinsäure und einer Kombination davon.

5. Formulierung nach Anspruch 1, wobei das Polymer oder die Mischung von Polymeren ausgewählt ist aus der Gruppe bestehend aus Hypromellose, Hydroxyethylcellulose, Hydroxypropylcellulose, Hydroxypropylmethylcellulose, Carboxymethylcellulose, Methylcellulose, Natriumcarboxymethylcellulose oder Salzen davon, Ethylcellulose, Carbomeren, Methacrylsäuren, Polyethylenoxiden (PEO) und einer Kombination davon.

6. Formulierung nach Anspruch 5, wobei das Polymer oder die Mischung von Polymeren Hydroxypropylmethylcellulose mit einer scheinbaren Viskosität im Bereich von 0,1-150 Pa·s (100-150.000 cP) (2 % in Wasser bei 20 °C) ist.

7. Formulierung nach einem der vorhergehenden Ansprüche, wobei die Formulierung Polymer mit kontrollierter Freisetzung im Bereich von 5 Gew.-% bis 30 Gew.-% der Formulierung umfasst.

8. Formulierung nach Anspruch 1, wobei der mindestens eine pharmazeutisch unbedenkliche Hilfsstoff ausgewählt ist aus der Gruppe bestehend aus Gummis, Füllstoffen, Fließhilfsmitteln, Schmiermitteln, Sprengmitteln, Verdünnungsmitteln, Bindemitteln, Schmiermitteln, Fließregulierungsmitteln und einer Kombination davon.

9. Formulierung nach Anspruch 1, umfassend bis zu 10 Gew.-% Antioxidans.

10. Formulierung nach Anspruch 9, wobei das Antioxidans gewählt ist aus Butylhydroxyanisol (BHA), Butylhydroxytoluol (BHT), Natriummetabisulfit, Natriumthiosulfat, Propylgallat, Ascorbinsäure und Cystein oder Kombinationen davon.

11. Formulierung nach einem der vorhergehenden Ansprüche, wobei die Formulierung optional eine funktionelle oder nicht funktionelle Beschichtung umfasst.

12. Formulierung nach einem der vorhergehenden Ansprüche, wobei die Formulierung ein Auflösungsfreisetzungsprofil *in vitro,* bei Messung unter Verwendung der USP-II-Methode, bei 75 U/min in 1000 ml, 0,1-N-HCl (pH 1,2), bei 37±0,5 °C, von 25 % bis 40 % nach 1 Stunde, 30 % bis 50 % nach 2 Stunden, 40 % bis 65 % nach 4 Stunden, 60 % bis 85 % nach 8 Stunden und nicht weniger als etwa 85 % nach 16 Stunden aufweist.

13. Verfahren zum Herstellen einer einschichtigen Tablette mit kontrollierter Freisetzung, die Drotaverin oder ein Salz davon umfasst, wobei das Verfahren Folgendes umfasst:
(a) Sieben und Trockenmischen von Drotaverin oder Salz davon mit einem Ansäuerungsmittel, Polymer und Hilfsstoff, um ein Arzneimittel-Hilfsstoff-Gemisch zu erhalten;
(b) Sieben und Mischen von extragranulären Inhaltsstoffen mit dem Arzneimittel-Hilfsstoff-Gemisch, um eine Formulierung zu erhalten;
(c) Komprimieren der Formulierung, um eine Tablette auszubilden.

14. Verfahren zum Herstellen einer ein- oder mehrschichtigen Tablette, die Drotaverin oder ein Salz davon umfasst, wobei das Verfahren Folgendes umfasst:
(a) Sieben und Trockenmischen von Drotaverin oder Salz davon mit einem Ansäuerungsmittel, Polymer und Hilfsstoff, um eine Trockenmischung zu erhalten;
(b) Granulieren der Trockenmischung mit einer Bindemittellösung, die mindestens Polyvinylpyrrolidon und Isopropylalkohol umfasst, um ein Granulat zu erhalten;
(c) Trocknen des Granulats, um einen erwünschten Trocknungsverlust des getrockneten Granulats zu erhalten;
(d) Mahlen des getrockneten Granulats, gefolgt von Sieben, um ein Granulat einer bestimmten Größe zu erhalten;
(e) Sieben von extragranulären Inhaltsstoffen, gefolgt von Mischen mit dem Granulat einer bestimmten Größe, um eine Formulierung zu erhalten; und
(f) Komprimieren der Formulierung, um eine Tablette auszubilden.

15. Formulierung nach einem der Ansprüche 1 bis 12 zur Verwendung bei der Behandlung mindestens eines Symptoms einer gastrointestinalen, biliären, urologischen oder gynäkologischen Störung, die durch spastische Zustände glatter Muskeln bei einem Subjekt gekennzeichnet ist, umfassend Verabreichen der Formulierung an das Subjekt.

## Revendications

1. Formulation comprenant une partie à libération contrôlée et une partie à libération immédiate, ladite formulation comprenant la drotavérine ou un sel de celle-ci, un polymère ou un mélange de polymères, et au moins un excipient acceptable pharmaceutiquement, ladite formulation comprenant au moins un agent acidifiant et de 0 à 10 % en poids d'antioxydants.

2. Formulation de la revendication 1, ladite formulation comprenant 10 à 300 mg de drotavérine ou un sel de celle-ci.

3. Formulation de la revendication 1 ou la revendication 2, dans laquelle le sel de drotavérine est le chlorhydrate de drotavérine.

4. Formulation de la revendication 1, dans laquelle ledit au moins un agent acidifiant est choisi dans le groupe constitué de l'acide citrique, de l'acide fumarique, de l'acide lactique, de l'acide maléique, de l'acide malique, de l'acide tartrique et d'une combinaison de ceux-ci.

5. Formulation de la revendication 1, dans laquelle le polymère ou le mélange de polymères est choisi dans le groupe constitué par l'hypromellose, l'hydroxyéthylcellulose, l'hydroxypropylcellulose, l'hydroxypropylméthylcellulose, la carboxyméthylcellulose, la méthylcellulose, la carboxyméthylcellulose sodique ou des sels de celle-ci, l'éthylcellulose, des carbomères, des acides méthacryliques, des oxydes de polyéthylène (PEO) et une combinaison de ceux-ci.

6. Formulation de la revendication 5, dans laquelle le polymère ou le mélange de polymères est de l'hydroxypropylméthylcellulose présentant une viscosité apparente comprise entre 0,1 et 150 Pa·s (100 à 150 000 cP) (2 % dans de l'eau à 20 °C).

7. Formulation de l'une quelconque des revendications précédentes, ladite formulation comprenant un polymère à libération contrôlée dans la plage de 5 % à 30 % (p/p) de la formulation.

8. Formulation de la revendication 1, dans laquelle l'au moins un excipient acceptable pharmaceutiquement est choisi dans le groupe constitué de gommes, de charges, de fluidifiants, de lubrifiants, de délitants, de diluants, de liants, de lubrifiants, d'agents de glissement et d'une combinaison de ceux-ci.

9. Formulation de la revendication 1, comprenant jusqu'à 10 % en poids d'antioxydant.

10. Formulation de la revendication 9, dans laquelle l'antioxydant est choisi parmi l'hydroxyanisole butylé (BHA), l'hydroxytoluène butylé (BHT), le métabisulfite de sodium, le thiosulfate de sodium, le gallate de propyle, l'acide ascorbique et la cystéine ou des combinaisons de ceux-ci.

11. Formulation de l'une quelconque des revendications précédentes, ladite formulation comprenant éventuellement un revêtement fonctionnel ou non fonctionnel.

12. Formulation de l'une quelconque des revendications précédentes, ladite formulation présentant un profil de libération par dissolution *in vitro,* lorsqu'il est mesuré par la méthode USP II, à 75 tr/min, dans 1000 ml de HCl 0,1 N (pH 1,2), à 37±0,5 °C, de 25 % à 40 % après 1 heure, de 30 % à 50 % après 2 heures, de 40 % à 65 % après 4 heures, de 60 % à 85 % après 8 heures et de pas moins d'environ 85 % après 16 heures.

13. Procédé de préparation d'un comprimé à libération contrôlée monocouche comprenant de la drotavérine ou un sel de celle-ci, le procédé comprenant :
(a) le tamisage et le mélange à sec de la drotavérine ou d'un sel de celle-ci avec un agent acidifiant, un polymère et un excipient pour obtenir un mélange médicament-excipient ;
(b) le tamisage et le mélange d'ingrédients extra-granulaires avec le mélange médicament-excipient pour obtenir une formulation ;
(c) la compression de la formulation pour former le comprimé.

14. Procédé de préparation d'un comprimé monocouche ou multicouche comprenant de la drotavérine ou un sel de celle-ci, le procédé comprenant :
(a) le tamisage et le mélange à sec de la drotavérine ou d'un sel de celle-ci avec un agent acidifiant, un polymère et un excipient pour obtenir un mélange sec ;
(b) la granulation du mélange sec avec une solution de liant comprenant au moins de la polyvinylpyrrolidone et de l'alcool isopropylique pour obtenir des granulés ;
(c) le séchage des granulés pour obtenir une perte à la dessiccation souhaitée des granulés séchés ;
(d) le broyage des granulés séchés suivi d'un tamisage pour obtenir des granulés de taille spécifique ;
(e) le tamisage d'ingrédients extra-granulaires suivi d'un mélange avec les granulés de taille spécifique pour obtenir une formulation ; et
(f) la compression de la formulation pour former le comprimé.

15. Formulation de l'une quelconque des revendications 1 à 12, destinée à être utilisée dans le traitement d'au moins un symptôme d'un trouble gastro-intestinal, biliaire, urologique ou gynécologique **caractérisé par** des états spastiques des muscles lisses chez un sujet, comprenant l'administration de la formulation au sujet.
